# EUROPEAN PATENT SPECIFICATION

(11) **EP 3 766 506 B1**
(45) Date of publication and mention of the grant of the patent: **11.09.2024**
(21) Application number: 19766786.8
(22) Date of filing: 30.01.2019
(51) Int. Cl.: A61K 35/30, A61K 35/44, A61P 9/00, A61P 25/28, C12N 5/079, C12N 5/0797

(54) **ANGIOGENESIS-INDUCING METHOD USING NEURAL STEM CELL**
ANGIOGENESE-INDUZIERENDES VERFAHREN MIT NEURALER STAMMZELLE
PROCÉDÉ D'INDUCTION DE L'ANGIOGENÈSE UTILISANT DES CELLULES SOUCHES NEURALES

(30) Priority: 16.03.2018 KR 20180031099; 29.01.2019 KR 20190011224
(43) Date of publication of application: 20.01.2021
(73) Proprietor: Medinno Inc., Gyeonggi-do 16419 (KR)
(72) Inventor: JOO, Kyeung Min, Seoul 08000 (KR); NAM, Hyun, Gimpo-si, Gyeonggi-do 10077 (KR)
(74) Representative: Dehns
(86) International application number: PCT/KR2019/001287
(87) International publication number: WO 2019/177269

(56) References cited:
- CN-A- 1 621 515
- KR-A- 20130 100 053
- KR-A- 20160 022 667
- KR-A- 20180 012 793
- US-A1- 2005 026 136
- US-A1- 2007 264 712
- US-A1- 2010 196 963
- US-A1- 2010 209 398
- US-A1- 2010 215 714
- US-A1- 2010 310 530
- SCHMIDT N O ET AL: "Vascular endothelial growth factor-stimulated cerebral microvascular endothelial cells mediate the recruitment of neural stem cells to the neurovascular niche", BRAIN RESEARCH, ELSEVIER, AMSTERDAM, NL, vol. 1268, 1 May 2009 (2009-05-01), pages 24 - 37, XP026038143, ISSN: 0006-8993, [retrieved on 20090310], DOI: 10.1016/J.BRAINRES.2009.02.065
- WILFRED A JEFFERIES ET AL: "Adjusting the compass: new insights into the role of angiogenesis in Alzheimer?s disease", ALZHEIMERS RES THER, BIOMED CENTRAL LTD, LONDON, UK, vol. 5, no. 6, 19 December 2013 (2013-12-19), pages 64, XP021193613, ISSN: 1758-9193, DOI: 10.1186/ALZRT230
- VAGNUCCI A H ET AL: "Alzheimer's disease and angiogenesis", THE LANCET, ELSEVIER, AMSTERDAM, NL, vol. 361, no. 9357, 15 February 2003 (2003-02-15), pages 605 - 608, XP004778573, ISSN: 0140-6736, DOI: 10.1016/S0140-6736(03)12521-4
- RODRÍGUEZ-JIMÉNEZ FRANCISCO JAVIER ET AL: "Hypoxia Causes Downregulation of Mismatch Repair System and Genomic Instability in Stem Cells", STEM CELLS, vol. 26, no. 8, 29 May 2008 (2008-05-29), pages 2052 - 2062, XP093002115, ISSN: 1066-5099, Retrieved from the Internet <URL:https://onlinelibrary.wiley.com/doi/full-xml/10.1634/stemcells.2007-1016> [retrieved on 20221127], DOI: 10.1634/stemcells.2007-1016
- LEI HAO; ZHONGMIN ZOU; HONG TIAN; YUBO ZHANG; HUCHUAN ZHOU; LEI LIU: "Stem Cell -Based Therapies for Ischemic Stroke", BIOMED RESEARCH INTERNATIONAL, vol. 2014, 468748, 26 February 2014 (2014-02-26), pages 1 - 18, XP055219763, ISSN: 2314-6133, DOI: 10.1155/2014/468748
- SASAN SHAREE GHOURICHAEE; JENNIE B LEACH: "The effect of hypoxia and laminin-rich substrates on the proliferative behavior of human neural stem cells", JOURNAL OF MATERIALS CHEMISTRY B , vol. 4, 25 March 2016 (2016-03-25), pages 3509 - 3514, XP055639077, ISSN: 2050-750X, DOI: 10.1039/C5TB02701B

## Description

### [Technical Field]

The present invention relates to neural stem cells for use in a method of treating, by inducing angiogenesis, stroke or spinal cord injury.

### [Background Art]

Stem cells are capable of self-renewal and differentiation into various cells, can be derived from an embryo or fetus. It is also known that resident stem cells in adult tissues can regenerate their own tissues. Mesenchymal stem cells are well known as adult stem cells and are the leading material for clinical researches worldwide.

Neural stem cells are stem cells that are important in treatment of degenerative neurological disorders because they are capable of self-proliferation and differentiation into neurons, astrocytes or oligodendrocytes. Neural stem cells were first identified in rodent, mice, and are known to exist in a specific region of the brain. Since then, it has been known that neural stem cells are present in the human brain, and human neural stem cells acquired from fetal brain tissue can be expanded *ex vivo* and have been used in clinical trials. Recently, it has been reported that neural stem cells exist in adult human brain tissue, and they can be preferably expanded by adherent culture, rather than sphere culture.

Angiogenesis refers to the growth of a new vessel, and a process mainly dependent on migration, proliferation, and formation of capillary endothelial cells. Angiogenesis may form a blood vessel under physiological and pathological conditions including wound healing or cancer. The regulation of angiogenesis is site- and stimulus-dependent, and may, in each case, involve a unique combination of regulatory molecules. During angiogenesis, endothelial cells rapidly proliferate, escaping from their stationary state.

Meanwhile, Matrigel (trade name for the product by BD Bioscience) is a protein complex extracted from Engelbreth-Holm-Swarm (EHS) mouse sarcoma cells, and contains extracellular matrix (ECM) components such as laminin, collagen and heparan sulfate proteoglycan, and growth factors such as a fibroblast growth factor (FGF), an epidermal growth factor (EFG), an insulin-like growth factor (IGF), a transforming growth factor-beta (TGF-β) and a platelet-derived growth factor (PDGF). The complex constituting Matrigel is used as a cell culture matrix by providing a complicated extracellular environment found in various tissues.

Matrigel has been used to reinforce cardiomyocytes and endothelial cells transplanted into an ischemic animal model, and since embryonic stem cells (ESCs) have self-renewability and pluripotency, Matrigel has been used for *ex vivo* culture of murine and human embryonic stem cells.

Therefore, the inventors confirmed from an experiment using Matrigel in which, among stem cells with commercial usefulness in medical substances and cosmetics having various functions, neural stem cells derived from human brain tissue secrete MCP-1 and Gro to have an angiogenesis-inducing effect.

US2007/264712A1 relates to methods and compositions for enhancing the growth of neural stem cells (NSCs), and refers to culturing NSCs under lowered oxygen conditions as compared to environmental oxygen conditions traditionally employed in cell culture techniques.

US2010/215714A1 relates to cells derived from postpartum tissue such as the umbilical cord and placenta, and refers to methods for their use to regenerate, repair, and improve neural tissue, and to improve behavior and neurological function in stroke patients.

US2010/196963A1 relates to a method of producing compositions including embryonic proteins, the method including culturing cells under hypoxic conditions on a biocompatible surface in vitro.

### [Disclosure]

As a result of the inventors conducting a study on functions of conventional neural stem cells, other than a known function, such as differentiation into neural cells, it was confirmed that neural stem cells derived from human brain tissue secrete MCP-1 and Gro, thereby obtaining an angiogenesis-inducing effect.

### [Technical Solution]

Embodiments of the present invention are described in the claims.

To attain the purpose of the present invention, the present invention provides neural stem cells for use in a method of treating, by inducing angiogenesis, stroke or spinal cord injury, said method comprising:
(a) culturing neural stem cells under a hypoxia condition by treating the neural stem cells with cobalt (II) chloride (CoCl₂); and
(b) injecting the cultured neural stem cells into a subject,

wherein step (b) includes injecting vascular endothelial cells along with the neural stem cells into a subject, and
wherein the cultured neural stem cells secrete monocyte chemoattractant protein-1 (MCP-1) or a growth-related oncogene (Gro).

In one embodiment of the present invention, the neural stem cells may be derived from human brain tissue.

In accordance with the present invention, the step (a) includes treating neural stem cells with CoCl₂.

In accordance with the present invention, the step (b) includes injecting vascular endothelial cells along with the neural stem cells into a subject.

In accordance with the present invention, the cultured neural stem cells secrete MCP-1 or Gro.

In yet another embodiment of the present invention, the CoCl₂ may have a concentration of 100 to 500 µM.

### [Advantageous Effects]

It was confirmed that neural stem cells derived from human brain tissue have an effect of forming blood vessels by secreting MCP-1 or Gro, and when CoCl₂ is applied to the neural stem cells, a hypoxia condition induces neural stem cells to increase secretion of MCP-1 and Gro, and thus it is expected that angiogenesis can be induced using human brain tissue-derived neural stem cells in accordance with the present invention.

### [Description of Drawings]

FIGS. 1A to 1C show the angiogenesis-inducing effect of neural stem cells through a Matrigel plug assay according to one embodiment of the present invention, wherein FIG. 1A is a diagram showing the outline of a Matrigel plug assay, FIG. 1B is a diagram showing the presence or absence of angiogenesis on day 3 after transplantation of a mixture of neural stem cells and vascular endothelial cells which is discerned by color due to penetration of red blood cells, and FIG. 1C is a diagram showing the histologic result of the Matrigel plug assay by H&E staining.
FIGS. 2A and 2B show qualitative and quantitative analysis of angiogenesis-related factors of neural stem cells using a cytokine array (FIG. 2A) and enzyme-linked immunosorbent assay (ELISA, FIG. 2B) according to one embodiment of the present invention.
FIG. 3 shows the angiogenesis inhibitory effect by MCP-1 and Groneutralizing antibodies according to one embodiment of the present invention.
FIG. 4 shows a signaling pathway in which angiogenesis-related expression of MCP-1 and Gro secreted under a hypoxia condition induced by CoCl₂ treatment in neural stem cells according to one embodiment of the present invention is increased.
FIG. 5 is a diagram illustrating an experimental design for confirming the level of MCP-1 and Groα secreted from neural stem cells, following induction of a hypoxia condition by treating CoCl₂ with various concentrations.
FIG. 6A shows results obtained by measuring the concentration of MCP-1 by ELISA following treatment of CoCl₂ with various concentrations.
FIG. 6B shows results obtained by measuring Groα concentrations by ELISA following treatment of CoCl₂ with various concentrations.
FIG. 7 is a diagram illustrating an experimental design for confirming the expression of MCP-1 and Groα secreted from neural stem cells following induction of a hypoxia condition by culturing the neural stem cells in a hypoxia chamber.
FIG. 8 shows the results obtained by measuring the concentrations of MCP-1 and Groα over time by ELISA following culture of neural stem cells in a hypoxia chamber.

### [Modes of the Invention]

The present invention provides neural stem cells for use in a method of treating, by inducing angiogenesis, stroke or spinal cord injury, said method comprising:
(a) culturing neural stem cells under a hypoxia condition by treating the neural stem cells with cobalt (II) chloride (CoCl₂); and
(b) injecting the cultured neural stem cells into a subject,

wherein step (b) includes injecting vascular endothelial cells along with the neural stem cells into a subject, and
wherein the cultured neural stem cells secrete monocyte chemoattractant protein-1 (MCP-1) or a growth-related oncogene (Gro).

The neural stem cells of the present invention may be derived from human brain tissue, but the present invention is not limited thereto.

According to the present invention, the subject refers to a target in need of induction of angiogenesis, and more specifically, a mammal such as a human or a non-human primate, a mouse, a rat, a dog, a cat, a horse, or a cow.

According to the present invention, the hypoxia condition may be a cell culture condition having an oxygen partial pressure of 1% to 5%, but the present invention is not limited thereto.

In accordance with the present invention, the step (a) includes treating neural stem cells with CoCl₂.

In accordance with the present invention, the step (b) includes injecting vascular endothelial cells along with the neural stem cells into a subject.

In addition, the cultured neural stem cells may induce formation of blood vessels by secreting MCP-1 or Gro, but the present invention is not limited thereto.

Here, the "monocyte chemoattractant protein-1 (MCP-1)" is a monocytic chemotactic factor and a protein of the chemokine β subfamily. MCP-1 has a potent chemotactic effect on monocytes, and exhibits effects on T lymphocytes, mast cells and basophilic leukocytes. Although MCP-1 is made by various types of cells (leukocytes, platelets, fibroblasts, endothelial cells and smooth muscle cells), it has highest specificity for monocytes and macrophages, and constitutes an activating stimulus as well as a chemotactic factor, resulting in induction of processes of forming multiple inflammatory factors (superoxides, arachidonic acid and derivatives, cytokines/chemokines) and amplification of phagocytosis.

In addition, the "growth-related oncogene (Gro)" is also referred to as melanoma growth stimulatory activity (MGSA), and includes three types of isoforms such as GROα (MGSAα, CXCL1), GROβ (MGSAβ, CXCL2) and GROγ (MGSAγ, CXCL3), which belong to CXC chemokines. The Gro includes its first two cysteine residues having a CXC structure and a glutamic acid-leucine-arginine (ELR) motif.

When CoCl₂ is applied to neural stem cells, a hypoxia condition may be induced and result in the increase of MCP-1 and Gro secretion, and in this case, the CoCl₂ concentration may be 100 to 500 µM. According to an exemplary embodiment of the present invention, the neural stem cells treated with CoCl₂ at a concentration of 500 µM for 24 hours or 100 µM and for 24 hours and then further cultured for 24 hours after replacement of the medium, are preferable because MCP-1 and Gro secretion are high, but the present invention is not limited to the concentration.

Through histological analysis using a Matrigel plug assay and H&E staining, the angiogenesis-inducing effect of neural stem cells was confirmed (see Examples 1 and 2).

The amounts of MCP-1 and Gro secreted from neural stem cells were confirmed using a cytokine assay and ELISA (see Example 3).

The angiogenesis-inducing effect by MCP-1 and Gro was confirmed using a neutralizing antibody against MCP-1 and Gro, respectively (see Example 4).

An increase in MCP-1 and Groα expression levels by CoCl₂ in neural stem cells were confirmed (see Example 5).

MCP-1 and Groα expression levels after neural stem cells are cultured in a hypoxia chamber were confirmed (see Example 6). Example 6 is not according to the invention and is present for illustration purposes only.

Hereinafter, to help in understanding the present invention, exemplary examples will be suggested. However, the following examples are merely provided to understand the present invention more easily, and not to limit the present invention.

### Example 1. Matrigel plug assay

Experiments for verifying angiogenesis capability were performed with experimental groups consisted of a Matrigel-only group, a human umbilical cord vein epithelial cell (HUVEC)-only group, an adult human multipotent neural cell (ahMNC)-only group, a fetal neural stem cell (fNSC) and HUVEC mixed group, and an ahMNC and HUVEC-mixed group.

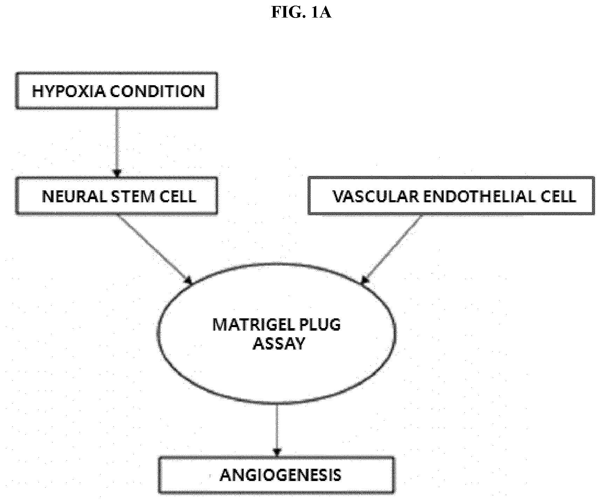

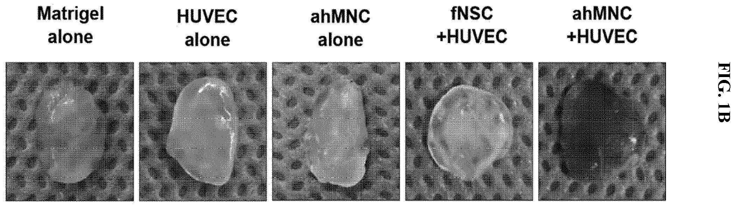

Neural stem cells (ahMNCs or a negative control fNSCs) and/or HUVECs were mixed with 200 µL of Matrigel at a 1:1 ratio such that a total cell number became 2 × 10⁶, and then transplanted subcutaneously into immunodeficient mice using a syringe. On day 3 after transplantation, the transplanted Matrigel was separated and then the presence or absence of blood vessel formation was discerned by color due to penetration of red blood cells. As a result, as shown in FIG. 1B, the ahMNC and HUVEC-mixed group was shown red.

Accordingly, from this result, blood vessels are formed by co-transplantation of neural stem cells and vascular endothelial cells.

### Example 2. Histological analysis

The Matrigel plugs of Example 1 were fixed with 4% PFA, and blocks was made. For H&E staining, 5-µm-thick tissue sections were made, placed on a slide, and then subjected to deparaffination, followed by H&E staining.

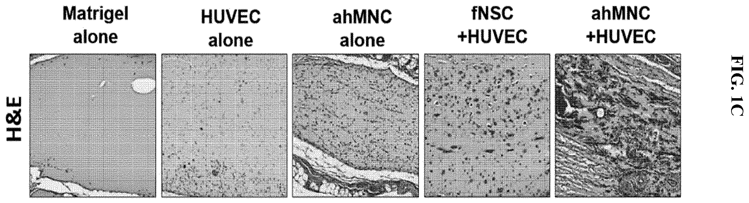

As a result, as shown in FIG. 1C, in the ahMNC and HUVEC-mixed group, the formation of blood vessels was confirmed.

Accordingly, from this result, it was seen that blood vessels are formed by co-transplantation of neural stem cells and vascular endothelial cells.

### Example 3. Quantification of MCP-1 and Gro using cytokine array and ELISA

To confirm the angiogenesis-inducing effect of neural stem cells, growth factors or cytokines secreted from neural stem cells were qualitatively and quantitatively analyzed.

Three different types of neural stem cells such as NS14-001TL, NS14-008TL and NS14-015TL were seeded in 100-mm dishes, cultured for 3 to 4 days to be approximately 70% confluency, washed twice with phosphate buffered saline (PBS), and then cultured in the DMEM/F12 medium for 24 hours. Afterward, a supernatant (conditioned medium) was harvested. A cytokine array was performed using a human cytokine array (RayBio Human Cytokine Antibody Array C Series 1000).

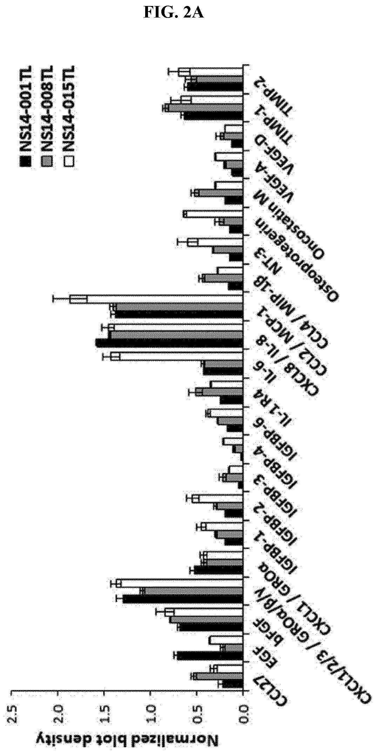

As a result, as shown in FIG. 2A, it was confirmed that various types of growth factors or cytokines such as MCP-1, Gro and IL-8 were secreted.

In addition, for quantification of MCP-1 and Gro found by the cytokine array, ELISA was performed with Quantikine ELISA kits (R&D Systems) using the recovered conditioned medium of the three different types of neural stem cells such as NS14-001TL, NS14-008TL and NS14-015TL.

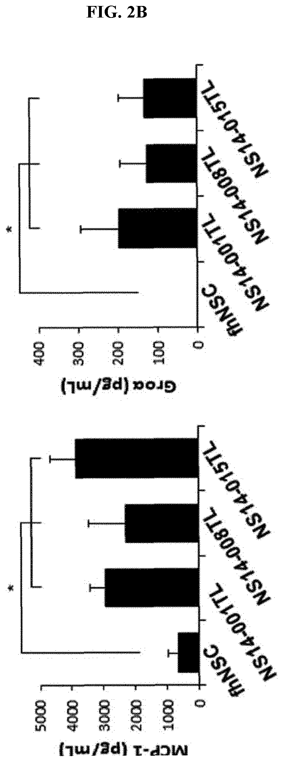

As a result, as shown in FIG. 2B, compared with a control, fNSC, the amounts of MCP-1 and Groα were increased in all of the three types of neural stem cells.

### Example 4. Confirmation of angiogenesis-inducing effect of MCP-1 and Gro

To confirm the angiogenesis-inducing effect of MCP-1 and Gro, a neutralizing antibody against each of MCP-1 and Gro was mixed with Matrigel and then transplanted to confirm angiogenesis inhibition.

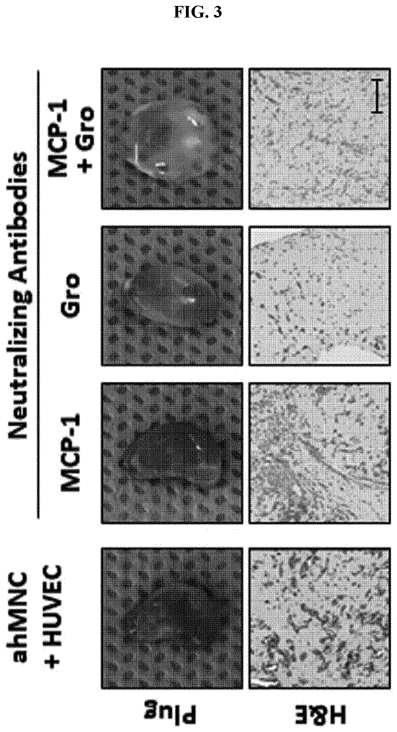

As a result, as shown in FIG. 3, when antibodies blocking the functions of MCP-1 and Gro were transplanted with Matrigel plugs, there was little angiogenesis. Moreover, when the antibodies blocking the functions of MCP-1 and Gro were mixed, it was confirmed that a synergistic angiogenesis inhibitory effect was exhibited.

### Example 5. Confirmation of MCP-1 and Gro expression levels by CoCl₂ treatment in neural stem cells

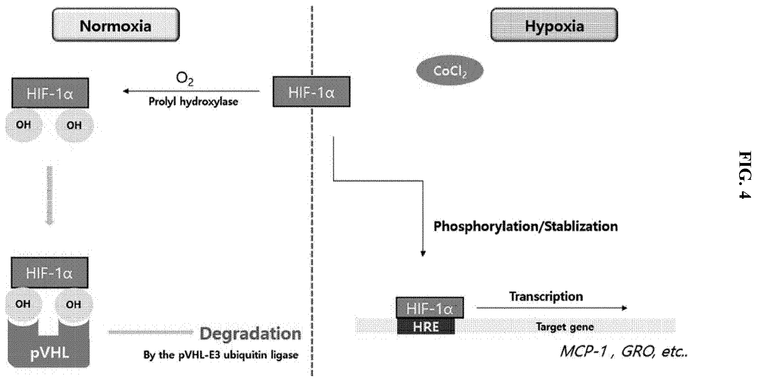

As shown in FIG. 4, when CoCl₂ was treated at various concentrations such as 100, 200, 300, 400 and 500 µM to reflect a hypoxia condition, the expression of MCP-1 and Gro increased by stabilizing HIF-1α, a transcription factor.

### 5-1. CoCl₂ treatment in neural stem cells

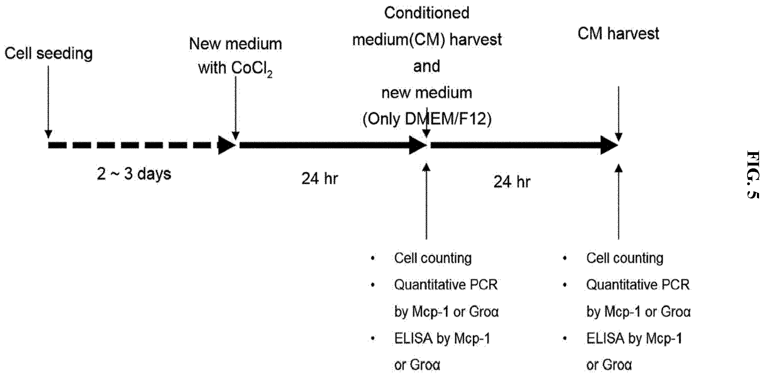

Human adult neural stem cells were cultured in a 100-mm culture dish for 2 to 3 days to reach approximately 70 to 80% confluency. Afterward, to reflect a hypoxia condition, CoCl₂ was treated at various concentrations, for example, 100, 200, 300, 400 and 500 µM, and after 24 hours, the conditioned medium was harvested and replaced with DMEM/F 12. After 24 hours, the conditioned medium was harvested and then analyzed. This process is schematically shown in FIG. 5.

### 5-2. Quantification of MCP-1 and Groa using ELISA

For the quantification of MCP-1 and Groα in the conditioned medium recovered in Example 5-1, ELISA was performed using the Quantikine ELISA kits (R&D Systems).

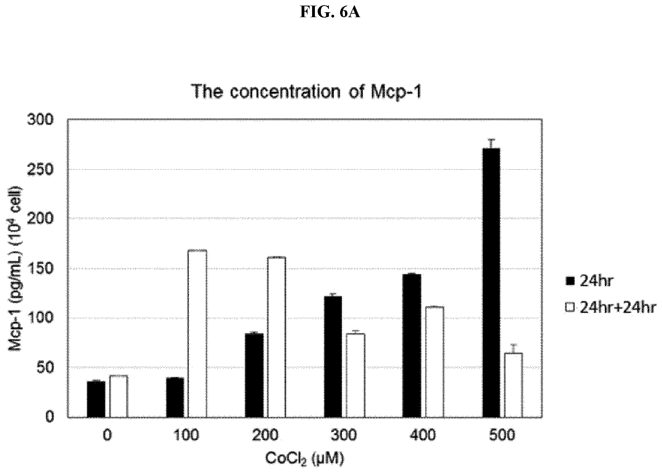

As a result, as shown in FIG. 6A, the amount of MCP-1 at 24 hours after CoCl₂ treatment, increased in proportion to the concentration of the treated CoCl₂. However, in the result of another 24 hours after the medium was replaced with DMEM/F12, the expression level of MCP-1 was highest in the case of treatment of 100 µM CoCl₂. When the CoCl₂ concentration was 300 µM or more, compared to treatment at 100 and 200 µM, the amount of MCP-1 was reduced.

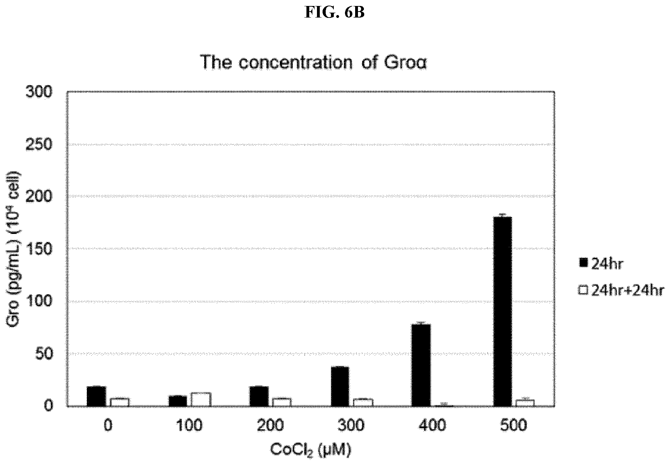

In addition, as shown in FIG. 6B, while the amount of Groα, at 24 hours after CoCl₂ treatment, increased in a concentration-dependent manner. The highest was observed when the CoCl₂ concentration was 500 µM. In the result of another 24 hours after the medium was replaced with DMEM/F12, it was confirmed that the amount of Groα was highest when 100 µM CoCl₂ was treated, but the Groα amount was reduced when the CoCl₂ concentration was 200 µM or more, compared to 100 µM.

### Example 6. Confirmation of MCP-1 and Groα expression levels in neural stem cells cultured in hypoxia chamber

### 6-1. Culture of neural stem cells in hypoxia chamber

To induce a hypoxia condition of neural stem cells, neural stem cells were cultured in a hypoxia chamber for 12, 24, 48 or 72 hours, and then a conditioned medium was recovered. The amounts of secreted MCP-1 and Groα were measured.

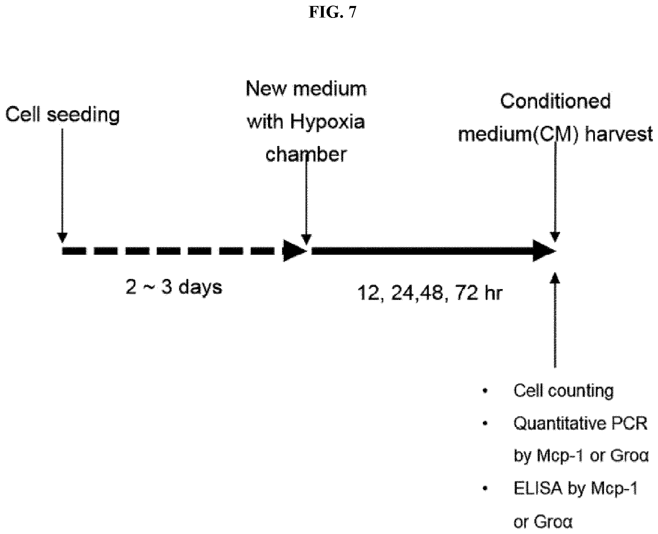

Human adult neural stem cells were cultured in a 100-mm culture dish to be 70 to 80% confluency for 2 to 3 days. Afterward, following replacement with a fresh medium, the cells were cultured in a hypoxia chamber for 12, 24, 48 or 72 hours, and then conditioned medium was recovered to perform ELISA. This procedure is schematically shown in FIG. 7.

### 6-2. Quantification of MCP-1 and Groα using ELISA

For quantification of MCP-1 and Groα in the conditioned medium recovered in Example 6-1, ELISA was performed using the Quantikine ELISA kits (R&D Systems).

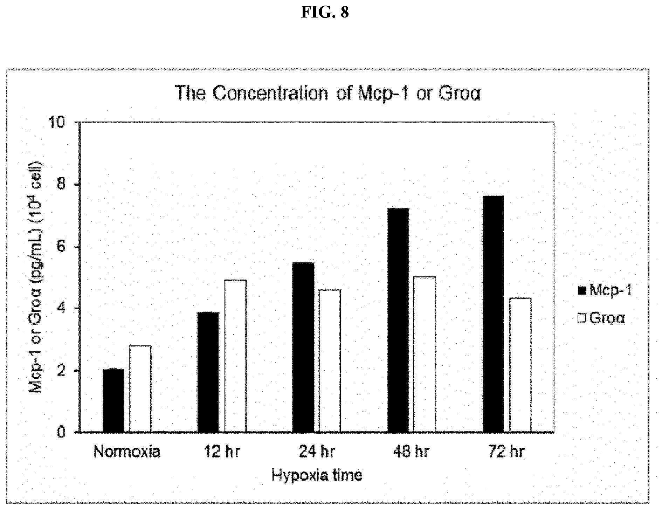

As a result, as shown in FIG. 8, while the expression of MCP-1 increased as the culture time passed, it was confirmed that the expression of Groα increased compared with a control, and remained almost constant after 48 hours in a hypoxic chamber.

### [Industrial Applicability]

When CoCl₂ is treated to neural stem cells in accordance with the present invention, it was confirmed that a hypoxia condition increases the amount of MCP-1 and Gro secretion. Human brain tissue-derived neural stem cells are cultured under a hypoxia condition so that they may be used to induce angiogenesis, and proteins or enzymes secreted from the neural stem cells may be used in angiogenesis-related developments such as purification of a conditioned medium and development of exosome. In addition, neural stem cells in accordance with the present invention are used as neural stem cell therapeutics against stroke or spinal cord injury, for which angiogenesis is known to be a recovery mechanism.

## Claims

1. Neural stem cells for use in a method of treating, by inducing angiogenesis, stroke or spinal cord injury, said method comprising:
(a) culturing neural stem cells under a hypoxia condition by treating the neural stem cells with cobalt (II) chloride (CoCl₂); and
(b) injecting the cultured neural stem cells into a subject,
wherein step (b) includes injecting vascular endothelial cells along with the neural stem cells into a subject, and
wherein the cultured neural stem cells secrete monocyte chemoattractant protein-1 (MCP-1) or a growth-related oncogene (Gro).

2. The neural stem cells for use of claim 1, wherein the neural stem cells are derived from human brain tissue.

3. The neural stem cells for use of claim 1 or claim 2, wherein the CoCl₂ is treated at a concentration of 100 to 500 µM.

## Patentansprüche

1. Neuronale Stammzellen zur Verwendung in einem Verfahren des Behandelns, durch Einleiten von Angiogenese, einer Schlaganfall- oder Rückenmarksverletzung, wobei das Verfahren umfasst:
(a) Kultivieren von neuronalen Stammzellen unter einer Hypoxie-Bedingung durch Behandeln der neuronalen Stammzellen mit Cobalt(II)-chlorid (CoCl₂); und
(b) Einspritzen der kultivierten neuronalen Stammzellen in ein Subjekt,
wobei Schritt (b) Einspritzen von vaskulären Endothelzellen zusammen mit den neuronalen Stammzellen in ein Subjekt einschließt, und
wobei die kultivierten neuronalen Stammzellen Monocyte Chemoattractant Protein-1 (MCP-1) oder ein wachstumsbezogenes Onkogen (Gro) absondern.

2. Neuronale Stammzellen zur Verwendung nach Anspruch 1, wobei die neuronalen Stammzellen von menschlichem Hirngewebe abgeleitet sind.

3. Neuronale Stammzellen zur Verwendung nach Anspruch 1 oder Anspruch 2, wobei das CoCl₂ bei einer Konzentration von 100 bis 500 µM behandelt wird.

## Revendications

1. Cellules souches neuronales pour utilisation dans un procédé de traitement, par induction d'angiogenèse, d'un accident vasculaire cérébral ou d'une lésion de la moelle épinière, ledit procédé comprenant :
(a) la mise en culture de cellules souches neuronales dans des conditions d'hypoxie par traitement des cellules souches neuronales avec du chlorure de cobalt (II) (CoCl₂); et
(b) l'injection des cellules souches neuronales de culture dans un sujet,
dans lesquelles l'étape (b) inclut l'injection de cellules endothéliales vasculaires conjointement avec les cellules souches neuronales dans un sujet, et
dans lesquelles les cellules souches neuronales de culture sécrètent la protéine 1 chimiotactique de monocytes (MCP-1) ou un oncogène lié à la croissance (Gro).

2. Cellules souches neuronales pour utilisation selon la revendication 1, dans lesquelles les cellules souches neuronales sont dérivées d'un tissu cérébral humain.

3. Cellules souches neuronales pour utilisation selon la revendication 1 ou la revendication 2, dans lesquelles le CoCl₂ est traité à une concentration de 100 à 500 µM.
